(19) Europäisches Patentamt / European Patent Office / Office européen des brevets

(11) **EP 3 511 722 A1**

(12) **EUROPEAN PATENT APPLICATION**

(43) Date of publication:
**17.07.2019 Bulletin 2019/29**

(51) Int Cl.:
***G01P 15/08*** (2006.01)   ***G01P 15/04*** (2006.01)
***A63B 60/46*** (2015.01)

(21) Application number: **18210193.1**

(22) Date of filing: **04.12.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.01.2018 AU 2018900065**

(71) Applicants:
• **Wall, Alexander**
  **West End, Queensland 4101 (AU)**

• **Michael, Dominic**
  **West End, Queensland 4101 (AU)**

(72) Inventors:
• **Wall, Alexander**
  **West End, Queensland 4101 (AU)**
• **Michael, Dominic**
  **West End, Queensland 4101 (AU)**

(74) Representative: **Cabinet Laurent & Charras
Le Contemporain
50 Chemin de la Bruyère
69574 Dardilly Cedex (FR)**

(54) **A VELOCITY AND COLLISION DETECTION SYSTEM**

(57) A velocity and collision detection system and particularly a system for detecting collisions and calculating the velocity of the object at the moment that an object strikes another.

Figure 1

**Description**

TECHNICAL FIELD

The present invention relates to a velocity and collision detection system and particularly a system for detecting collisions and calculating the velocity of the object at the moment that an object strikes another.

BACKGROUND ART

In sports, particularly sports that are broadcast on television, maintaining view engagement and interest is important. In recent times, broadcasters have increased the amount of information that they have provided to the viewers particularly in relation to athlete performance and/or statistics.

In the past, it has been difficult to ascertain statistics relating to bat speeds and ball speeds in balls using these implements. Conventionally, most speeds of this nature are calculated using a number of cameras to calculate position of the bat or ball over short period of time and extrapolating the speed of the bat or ball or using other devices such as radar guns and the like.

It will be clearly understood that, if a prior art publication is referred to herein, this reference does not constitute an admission that the publication forms part of the common general knowledge in the art in Australia or in any other country.

SUMMARY OF INVENTION

The present invention is directed to a velocity and collision detection system, which may at least partially overcome at least one of the abovementioned disadvantages or provide the consumer with a useful or commercial choice.

With the foregoing in view, the present invention in one form, resides broadly in a velocity and collision detection system including
an inertial measurement unit having

at least one accelerometer to collect accelerometer raw data,

at least one gyroscope to collect gyroscope raw data,

at least one transmitter to transmit the accelerometer raw data and the gyroscope raw data, and

at least one power source to power the at least one transmitter,

the inertial measurement unit configured to be mounted relative to a moving object to collect raw data in relation to the moving object, and
a raw data processor to receive the accelerometer raw data and the gyroscope raw data from the inertial measurement unit, process the accelerometer raw data and the gyroscope raw data to detect at least one movement event of the object to which the inertial measurement unit is mounted so that when a movement event is detected, at least one movement parameter of the moving object is calculated.

In an alternative form, the present invention in one form, resides broadly in a velocity and collision detection method including
providing an inertial measurement unit having at least one accelerometer to collect accelerometer raw data, at least one gyroscope to collect gyroscope raw data, at least one transmitter to transmit the accelerometer raw data and the gyroscope raw data, and at least one power source to power the at least one transmitter,
mounting the inertial measurement unit relative to a moving object to collect raw data in relation to the moving object,
receiving the accelerometer raw data and the gyroscope raw data from the inertial measurement unit at a raw data processor,
processing the accelerometer raw data and the gyroscope raw data to detect at least one movement event of the object to which the inertial measurement unit is mounted, and
calculating at least one movement parameter of the moving object when a movement event is detected.

Preferably the inertial measurement unit will be mounted to the moving object at or immediately adjacent to point of interest such as an impact zone or point of movement rather than remotely from the point of interest such as an impact zone or point of movement. Locating the inertial measurement unit remotely from the point of interest such as an impact zone or point of movement requires calculation of the at least one movement parameter at the point of interest by extrapolating parameters such as the angle, acceleration, and distance to the impact zone based on the raw data which introduces variables or calculation errors due to assumptions. For example, it is very difficult to ascertain the exact impact or hit point of a ball on a bat using an inertial measurements unit mounted to the handle of the bat.

In a preferred form, the velocity and collision detection system and method will further include the raw data processor undertaking a collision detection step wherein a collision is deemed to have occurred if there is a sudden acceleration change above a predetermined threshold between the raw data at a first time step compared to the raw data at an immediately subsequent time step.

If a collision is deemed to have occurred, the system and method of the present invention will preferably return the velocity of the object calculated at the first time step as a collision velocity.

In an alternative aspect, the present invention resides in an inertial measurement unit for use in a velocity and collision detection system, the inertial measurement unit having at least one accelerometer to collect accelerometer raw data, at least one gyroscope to collect gyroscope raw data, at least one transmitter to transmit the accelerometer raw data and the gyroscope raw data, and at least one power source to power the at least one transmitter, the inertial measurement unit configured to be mounted relative to a moving object to collect raw data in relation to the moving object.

Without wishing to be limited by theory, the present invention is based upon determining an object's acceleration and then integrating over time to obtain the velocity of the object. Integrating again, the position of the object can be determined.

The inertial measurement unit of the present invention will preferably be mounted relative to an object and the object will then be moved. The object can have virtually any form. The object will typically be one that a user will want to measure the velocity of. Example types of objects to which the inertial measurement unit of the present invention could be attached include sporting equipment such as a bat, racket or similar or a ball or other device such as a discus or javelin for example or the inertial measurement unit may be attached relative to a body part of a user such as a pitcher's arm or a bowler's arm or a runner's leg for example. Basically, the inertial measurement unit may be attached to any implement they user may want to measure the velocity of.

The at least one movement parameter will preferably be or include a parameter relating to the movement of the object. Parameters may include real time acceleration, real time angular acceleration, real time velocity and/or real time angular velocity or components f these in any one or more axes.

The at least one parameter may include an angular measurement or deviation instead of just velocity. An example of this is hit/ impact efficiency by measuring a deviation of a bat angle at point of impact. A more efficient shot would have less angle deviation thus putting more energy into the shot.

An alternative use case could be lean angle on a mountain bike through a corner.

The data may also be used to create a 3D path of an object (bat swing, arm, ball, object.

In a particularly preferred embodiment, the inertial measurement unit will be mounted relative to the object close to, if not in all at an impact zone or position, particularly in applications where a bat or racket is used to hit the ball. Location in a representative position is particularly important for the accuracy of the calculation of the velocity as it will normally be the velocity of the object in the representative position which is important. This will typically create issues with the robustness of the inertial measurement unit in order to withstand greater acceleration and/or impacts which may be occasioned in the representative position. It is to be noted that the prior art devices are rarely located in or immediately adjacent to the representative position such as an impact zone or hit point and are typically located remotely from the representative position such as the impact zone or hit point, which requires extrapolation or approximation which decreases the accuracy of the velocity measurement or calculation.

The inertial measurement unit of the preferred embodiment will typically be mounted relative to the object on an opposite side of the object to an impact position or hit point. In some embodiments, the inertial measurement unit may be provided at least partially, and sometimes wholly within the object.

The inertial measurement unit of a preferred embodiment will typically transmit collected raw data over a wireless communication link. One or more components of the inertial measurement unit may be responsible for packetizing the collected raw data prior to transmission.

The collected raw data may be adjusted or undergo some preliminary manipulation prior to transmission. For example, it is preferred that the collected raw data be manipulated to compensate for any drift which may occur in any one or more of the data collection components of the inertial measurement unit prior to transmission.

As will be explained further below, it is preferred that the raw data is collected on multiple axes (X, Y, Z).

The raw data (which includes the data which has been manipulated for compensation of drift) is typically sent or transmitted from the inertial measurement unit on a radio frequency but any frequency (including a non-radio frequency) could be used. A particularly preferred embodiment of the present invention transmits on approximately 900 MHz.

Alternative transmission platforms could be used such as Bluetooth for example which may save some weight and space given the need of a smaller antenna and/or a smaller battery, but this comes with ancillary issues such as the need for a Bluetooth relay located not be more than a few metres away, in order to be able to transmit the data over a reasonable distance. Data throughput is also quite limited via Bluetooth, resulting in sampling rates being either slower or data processing locally rather than after transmission.

The raw data is typically collected over a number of time steps or frames. Any length of time step or frame can be used. A particularly preferred frequency of collection of raw data is preferably approximately 1000 Hz.

The inertial measurement unit of the preferred embodiment includes at least one accelerometer. Any type of accelerometer may be used and any number of accelerometers may be used. For example, a number of accelerometers may be used to collect raw data in relation to each axis separately but a multiaxis accelerometer is preferred.

The inertial measurement unit of the preferred embodiment includes at least one gyroscope. Any type of gyroscope may be used and any number of gyroscopes may be used. For example, a number of gyroscopes may be used to collect raw data in relation to each axis separately but a multiaxis gyroscope is preferred.

Each of the at least one accelerometer is and the at least one gyroscopes will preferably both be provided on or relative to a printed circuit board assembly.

The inertial measurement unit of a preferred embodiment includes at least one accelerometer to detect and preferably measure linear acceleration and at least one gyroscope to detect and preferably measure angular acceleration. Detection and measurement will normally be measured relative to at least one axis and preferably relative to at least three axes, wherein a first axis is orthogonal to a second axis which are both orthogonal to a third axis.

Gyroscopes are frequently combined with accelerometers (acceleration sensors) for more robust direction-sensing and motion-sensing. A MEMS (microelectrical-mechanical system) gyroscope is a device that is used for measuring orientation and is a preferred gyroscope according to the present invention. Accelerometers can perform a similar function when they are stationary by measuring the components on each axis of Earth's gravitational field. However, if the accelerometer is experiencing acceleration other than gravity it will not be able to distinguish and consequently will not be able to determine orientation. This is where using at least one accelerometer in combination with at least one gyroscope becomes useful.

An accelerometer is a device that measures proper acceleration, being the acceleration (or rate of change of velocity) of a body in its own instantaneous rest frame, is not the same as coordinate acceleration, being the acceleration in a fixed coordinate system. An accelerometer at rest on the surface of the Earth will measure an acceleration due to Earth's gravity, straight upwards (by definition) of $g \approx 9.81$ m/s2. By contrast, accelerometers in free fall (falling toward the centre of the Earth at a rate of about 9.81 m/s2) will measure zero. Therefore, the effects of gravity must be accounted for when using the raw data from an accelerometer.

Single- and multi-axis models of accelerometer are available to detect magnitude and direction of the proper acceleration, as a vector quantity, and can be used to sense orientation (because direction of weight changes), coordinate acceleration, vibration, shock, and falling in a resistive medium (a case where the proper acceleration changes, since it starts at zero, then increases). Micro-machined microelectrical-mechanical systems (MEMS) accelerometers are increasingly preferred to detect the position of the device, the acceleration of the object or collect data for further processing.

A gyroscope is a device used for measuring or maintaining orientation and angular velocity. A number of single axis MEMS-based gyroscopes (one for detecting angular acceleration relative to each of a number of axes) may be used but a multi-axis MEMS-based gyroscope will preferably be used in in the inertial measurement unit of the preferred embodiment.

Together the at least one accelerometer and the at least one gyroscope will preferably provide 6 component motion sensing; acceleration for X, Y, and Z movement, both angular acceleration and linear acceleration.

Newer MEMS-based inertial measurement units incorporate up to all nine axes of sensing in a single integrated circuit package, providing inexpensive and widely available motion sensing. These types of devices may be used in the present invention.

The inertial measurement unit of the preferred embodiment also includes at least one transmitter. As mentioned above, it is preferred that the transmitter be or include a wireless transmitter and preferably, the transmitter will transmit on a radio frequency. The at least one transmitter is preferably associated with a printed circuit board assembly, typically the same printed circuit board assembly that the at least one accelerometer and at least one gyroscope are associated with in order to receive raw data from the at least one accelerometer and at least one gyroscope.

The printed circuit board assembly will preferably also mount at least one magnetometer. The magnetometer may assist with ascertaining the direction or heading of the object and/or its movement.

The printed circuit board assembly will also typically mount a microcontroller or processor in order to control operation of the components of the inertial measurement unit as well as to preferably apply the compensation for drift prior to transmission of the raw data.

The inertial measurement unit will normally include at least one power source, typically at least one battery. Any type and any number of batteries can be used but preferably, the battery will be relatively small in size, typically rechargeable, and preferably rechargeable in situ (although removal may be possible if this is preferred). A particularly preferred type of battery is a lithium polymer battery or more correctly, at least one lithium ion polymer battery. The battery may be charged by any mechanism but a wireless form of charging such as induction charging is preferred as this means that the housing can remain substantially sealed.

At least one resilient member or layer is preferably placed between the battery and the enclosure to protect the battery from any impacts and allow for expansion of the battery cell that can occur over time. The inertial measurement unit of the preferred embodiment will typically include at least one indicator light in order to indicate the operational status of the inertial measurement unit and/or any one or more of the components of the inertial measurement unit. Preferably, the at least one indicator light is provided on or relative to the printed circuit board assembly. At least one LED is preferred. A light pipe or similar structure is typically provided relative to the indicator light and the housing of the inertial measurement unit in order to transmit the light to the surface of the housing in order to be visible.

At least one switch is typically provided in relation to the inertial measurement unit to turn the unit on and off. Any type of switch may be used. Although a physical switch could be used, it is preferred that the switch be located inside the housing and operated using a magnet or similar which will mean that the housing can then be a closed housing.

The inertial measurement unit will typically be provided with an external housing to enclose the components of the inertial measurement unit. Typically, the housing will be a 2 part housing including a base, relative to which the preferred printed circuit board assembly is provided and a cover. The housing will normally be manufactured from a robust material and a plastic material is particularly preferred. The housing will normally be provided with at least one location to have branding applied to the exterior of the housing in order to allow the inertial measurement unit to be branded appropriately for the manufacturer or provider of the inertial measurement unit, and/or have team colours or branding applied thereto.

The inertial measurement unit is attached to or relative to an object which will be moved and normally, will be moved at a relatively high velocity and/or undergo large impacts. For this reason, the attachment mechanism used to attach the inertial measurement unit relative to the object will need to be extremely robust. Any type of attachment mechanism may be used. In the simplest form, an adhesive strip may be used to attach the inertial measurement unit directly to the object. If an adhesive strip is used, will normally be double sided and preferably thick enough to adjust to any curvature of the object if necessary.

Due to the location of the inertial measurement unit relative to the object in the preferred embodiment, the inertial measurement unit may be subjected to large forces, particularly due to impact. Unless precautions are taken, these large forces can result in the detachment of components from the preferred printed circuit board assembly which results in loss of function for the inertial measurement unit. Therefore, the inertial measurement unit will typically include at least one shock absorption system. According to a preferred embodiment, a foamed material or other resilient padding for example is preferably provided between the housing and the printed circuit board assembly and/or any components mounted to the printed circuit board assembly.

In a particularly preferred embodiment, the printed circuit board assembly and the components mounted thereto will preferably be potted (the process of filling in an assembly with a solid or gelatinous compound for resistance to shock or vibration). Any one or more thermosetting plastics or silicone rubber gels may be used for the potting. Preferably, a portion of the housing will be used as a mould for the potting and preferably, that portion of the housing will have a number of sidewalls which together define a containing portion into which the preferred printed circuit board assembly and the components mounted thereto will be placed before the application of the compound to pot the components.

One or more conformal coatings may be used but potting is a preferred method of shock absorption or buffering the printed circuit board assembly and components mounted thereto from the shock which may be occasioned through impacts to the object.

In the present invention, the system preferably uses calibration data to compensate for gyroscope drift. There are two main methods for compensating for drift.

Arguably the best method is to zero the gyroscope on a regular basis. Zeroing the gyro will reset the drift. The problem with this is that a gyroscope can only be zeroed when it is stationary. This means that movement needs to cease for a period of time (it can take a few seconds to complete the operation) to zero the gyroscope before continuing on with measurements.

The other main method which is more practically applicable is to continually correct the raw data collected for the drift. In order to do this, the drift rate needs to be measured over as long a period of time as possible. In order to measure the drift rate, the gyroscope is kept as stationary as possible for as long as possible. Checking the gyroscope reading at the end of the period ascertains how much the gyroscope has drifted over the time period allowing division to determine the drift amount for each individual raw data sample. It is then relatively simple to subtract the drift amount from each and every raw data sample.

Drift is not constant, but averaging over a large period of time will help negate any instantaneous ill effects but individual samples still have a margin of error associated with the subtracted drift value. It should however be minimal.

In practice a combination of both above strategies is the best, namely continually subtracting drift from the measurements and zeroing whenever it is possible to do so.

Therefore, the inertial measurement unit will be responsible for collecting the raw data from the components on board the inertial measurement unit (at least one accelerometer, at least on gyroscope and at least one magnetometer), compensate for drift in any one or more of the components, packetize the compensated raw data and transmit the compensated raw data, preferably over a radio frequency such as 900mHz, to a processor to be processed further.

The inertial measurement unit, system and method of the invention is preferably capable of calculating the velocity of an object at any time and only in some preferred situations will the system and method return a velocity at impact as it will only be in certain situations that the impact or collision velocity is of interest. In other circumstances, it may be that the velocity of a user's arm or leg is important for example in bowling or in a kicking sport and there may be no impact at all. The inertial measurement unit, system and method of the invention can be used to establish the velocity of an object or part of an object at any time.

To capture the velocity at impact time, it is necessary to firstly characterise how a meaningful impact can be detected during a cricket game using the device's pre-processed inertial data. Pre-processed in this case means the acceleration and gyroscope data have been filtered by the velocity acquisition algorithm. It also means any gaps in the raw data would have been filled based on a prediction model.

In order to detect relevant impacts to a ball for example as it strikes a bat, the bat moving direction is assessed first. The algorithm will only detect impacts if the bat is moving towards the ball, which means the acceleration must be negative along the z-axis and positive along the x based on the coordinates system.

Once that condition is met, a collision is detected if a sudden acceleration change reaches a heuristically-defined threshold between two samples. The velocity relative to the first sample is then returned, suggesting that was the speed of the bat immediately before the impact. A hysteresis factor is added to the threshold to avoid multiple consecutive detections.

According to a preferred embodiment of the present invention, the inertial measurement unit of the preferred embodiment will collect raw data from the at least one accelerometer and the at least one gyroscope, that raw data will then be manipulated to compensate for any drift in the at least one accelerometer in the at least one gyroscope and then the raw data will be packetised and transmitted to a remotely located processor, generally a software application in order to use the raw data to calculate the velocity of movement of the object. Typically, the at least one gyroscope will collect or detect data in relation to angular acceleration and the at least one accelerometer will collect or detect data in relation to linear acceleration.

According to a preferred embodiment, the raw data collected by the at least one accelerometer and at least one gyroscope is subjected to a prediction model based on estimation theory. The raw data collected regarding acceleration will often be associated with one or more random components such as noise for example. A "filter" is used to adjust the raw data to account for the bias of the components in the inertial measurement unit that collect the data for example, the Delta angle bias and the Delta velocity bias.

A preferred filter for use in the preferred embodiment is an Extended Kalman Filter (EKF) which is a non-linear version of a Kalman filter which linear rises about an estimate of the current mean and covariance.

The system of the present invention preferably uses a state vector prediction every time the raw data is received from the inertial measurement unit. The aim with using the state vector prediction model is to predict the state vector containing at least some of:

- the quaternions (4 parameter attitude representation);

- velocity (integrating the acceleration data once will give velocity information);

- position (integrating the acceleration data twice will give position information); and

- inertial measurement unit bias estimates.

The system of the present invention uses the state vector prediction model to predict the state vector forward in time using the inertial measurement unit raw data as the input into the model.

The inertial measurement bias state estimates are preferably not modified in the modelling process but are taken into account.

Typically, the process of the preferred embodiment will be used to integrate an angular velocity or rate vector and/or an acceleration vector for each time step or frame in order to calculate a Delta angle vector and the Delta velocity vector.

The Delta angle vector can then preferably be converted to a Delta quaternion set to obtain the quaternions (4 parameter attitude representation).

The quaternions can then preferably be used to convert to a rotation matrix in order to describe the rotation of the object relative to the Earth frame. The rotation matrix can then preferably be used in association with the Delta velocity vector and a gravity vector over time in order to determine velocity information and using trapezoidal integration, to determine position information relating to the object.

The system of the preferred embodiment can then calculate the speed (velocity) of the object as the magnitude of the estimated velocity vector that has been calculated. This is typically done only one in every ten or so frames (time steps) due to the computational load required to ascertain whether the solution from the prediction model is valid or not.

If the solution is not valid the filter and calculated velocity is reset and if the solution is valid, the system then moves to determine whether an impact is detected or not. If an impact is detected, then the velocity from the time step or frame immediately prior to the impact detection is output as the collision velocity.

In order to calculate speed as the magnitude of the estimated velocity vector, the system will preferably correct acceleration and angular rate data using learned biases. The system then typically uses the corrected data to calculate the g loading along the object's X and Z axes called 'gx' and 'gz' respectively, and the magnitude of the combined gx and gz magnitude called 'g_level'. The g loading is acceleration divided by gravity. The system then detect object movement events, for example bat swing events using gx and gz to detect the combination of angular and centripetal acceleration associated with that type of motion. The detection of a movement event will normally require g_level to exceed a threshold. A higher g_level threshold can be used if a swing is not declared.

If a swing event is not declared, then a non-swing event is declared. The system then preferably counts the number of swing and non-swing events.

The system will then preferably declare a swing when the required swing event count is exceeded, zero the non-swing event counter and zero the recorded peak speed.

The system will typically cancel the swing declaration when the required non-swing event count is exceeded, zero the swing event counter, set the impact declaration to false and copy the recorded peak speed to the reported peak speed.

If a swing is declared and g_level exceeds a threshold, the system will declare an impact and will output the velocity from the time step or frame immediately prior to the impact detection as the collision velocity.

When a swing is declared and no impact has been declared, the system will normally record the peak speed and may output this peak speed.

As mentioned above, zeroing to correct for drift is desirable if possible. The system of the present invention will preferably reset the filter is the object is still enough to reset the filter. Preferably the system will correct the acceleration and angular rate data using learned biases. The system will then preferably use the magnitude of the angular rate and acceleration vector to perform a motion check and detect if the object is still enough to reset the filter. If the check fails, the system will normally exit the reset and not clear the 'filter reset required' status. If the check passes, the system will normally clear the 'filter reset required' status and continue.

If a bias reset has been requested according to the above check, the system will preferably zero the IMU delta angle and delta velocity bias states. This is normally only requested during the first filter cycle.

The system will then preferably use the measured acceleration estimate the direction of gravity in the object's frame of reference and use that to set the initial angular orientation assuming zero yaw. The system also sets the velocity and position states to zero.

If no bias reset has been requested, according to the above check, the system will preferably record the variances for the IMU delta angle and delta velocity bias states. The system then preferably zeros the covariance matrix.

The system then preferably sets the variances to parameter specified values. If no bias reset has been requested, the system will preferably set the variances for the delta angle and delta velocity states to the value prior to zeroing the covariance matrix.

The estimated growth in state error over time is preferably performed by the 'covariance prediction' operation which uses a non-additive process noise. A number of tuneable parameters are normally provided to control the growth in covariance between measurements. These tuneable parameters include the IMU measurement noise and IMU bias process noise.

When the magnitude of the acceleration vector is below a threshold for a minimum continuous time interval, a zero velocity and zero position assumption is preferably used to prevent the inertial navigation from drifting. Different acceleration and time interval thresholds are normally used for position and velocity. These use a zero velocity or position as an observation vector in the standard EKF state and covariance update equations used in the system calculations. Each axis of the observation vector is typically treated as a separate measurement using a technique called 'sequential fusion' to improve computational efficiency.

A statistical confidence check is applied to each measurement axis and if any axis fails, a test fail is preferably reported and the measurement vector is not used.

If the fuse velocity or fuse position reports a continuous fail for longer than a specified interval, the acceleration magnitude is within specified limits and velocity fusion is active, then a filter reset is typically requested. This enables the filter to recover from the loss of navigation caused by the large acceleration events associated with impact, for example with the ground or a ball.

If the prediction model solution is valid after the statistical confidence check is applied to each measurement axis, then the velocity is calculated.

The system also moves to determine whether an impact has been declared. If an impact has been declared, then the velocity at the immediately preceding frame or time step is output as the collision velocity.

The IMU, system and method of the present invention therefore provide a far more accurate mechanism for calculating the velocity of an object, detecting collisions and calculating the velocity of the object at the moment of the collision than available in the prior art.

Any of the features described herein can be combined in any combination with any one or more of the other features described herein within the scope of the invention.

The reference to any prior art in this specification is not, and should not be taken as an acknowledgement or any form of suggestion that the prior art forms part of the common general knowledge.

BRIEF DESCRIPTION OF DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of the Invention in any way. The Detailed Description will make reference to a number of drawings as follows:

Figure 1 is an isometric view from the rear side of a cricket bat showing a sensor module according to a preferred embodiment of the invention attached thereto.

Figure 2 is an isometric exploded view of the configuration illustrated in Figure 1.

Figure 3 is an elevation view from the rear of the configuration illustrated in Figure 1.

Figure 4 is an elevation view from the right side of the configuration illustrated in Figure 1.

Figure 5 is an elevation view from the left side of the configuration illustrated in Figure 1.

Figure 6 is a view from the bottom of the configuration illustrated in Figure 1.

Figure 7 is a view from the top of the configuration illustrated in Figure 1.

Figure 8 is an isometric view of a sensor module according to a preferred embodiment of the invention.

Figure 9 is an exploded isometric view of the sensor module illustrated in Figure 8.

Figure 10 is a view from the rear of the sensor module illustrated in Figure 8.

Figure 11 is a view from the right side of the sensor module illustrated in Figure 8.

Figure 12 is a view from the left side of the sensor module illustrated in Figure 8.

Figure 13 is a view from the bottom of the sensor module illustrated in Figure 8.

Figure 14 is a view from the top of the sensor module illustrated in Figure 8.

Figure 15 is a flowchart of information flow through the system of a preferred embodiment of the present invention.

Figure 16 is a flowchart showing the prediction model used according to a preferred embodiment of the present invention.

Figure 17 is a flowchart showing the functions undertaken in the predict state vector step from the prediction model illustrated in Figure 16.

Figure 18 is a flowchart of the calculation of the delta angle vector in the single frame integration of the predict state vector step illustrated in Figure 17.

Figure 19 is a flowchart of the calculation of the delta velocity vector in the single frame integration of the predict state vector step illustrated in Figure 17.

Figure 20 is a flowchart of the inertial navigation calculation of the predict state vector step illustrated in Figure 17.

Figure 21 is an illustration of the bat axis system definition for use in the preferred embodiment.

DESCRIPTION OF EMBODIMENTS

According to a particularly preferred embodiment of the present invention, a velocity and collision detection system is provided.

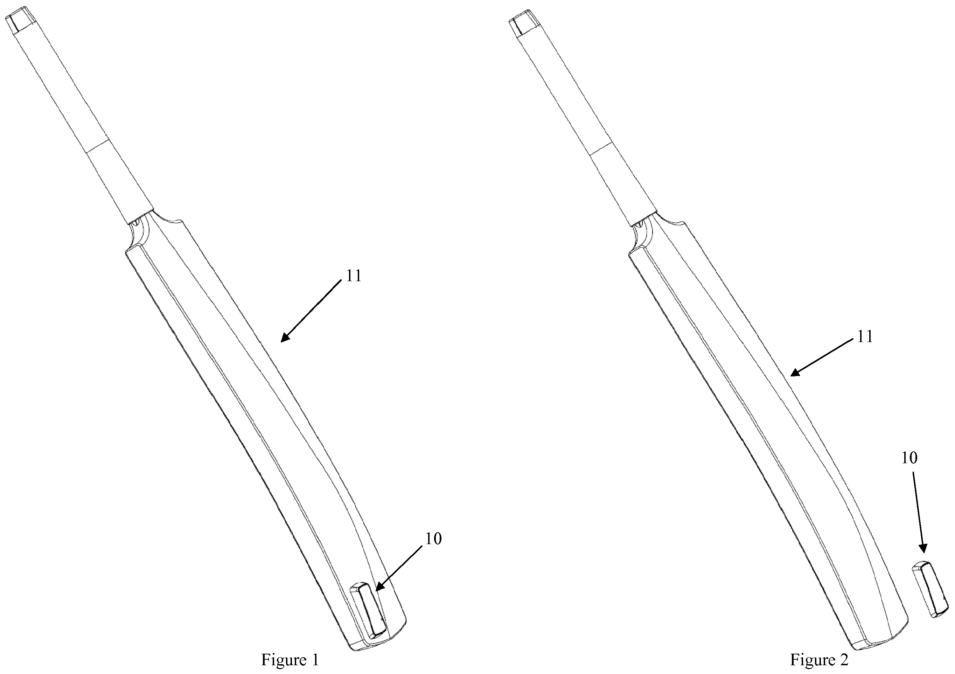

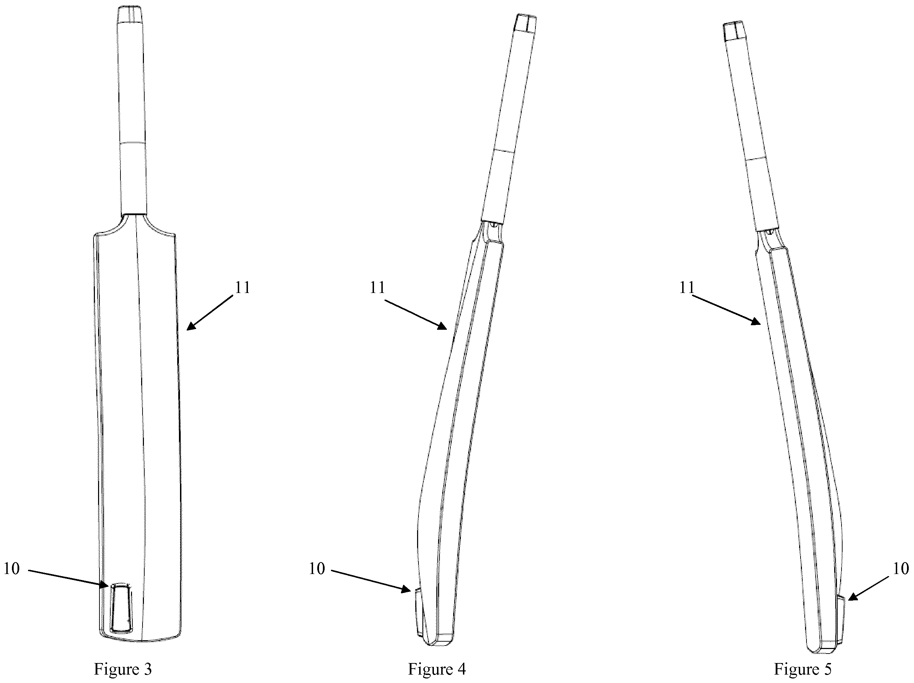

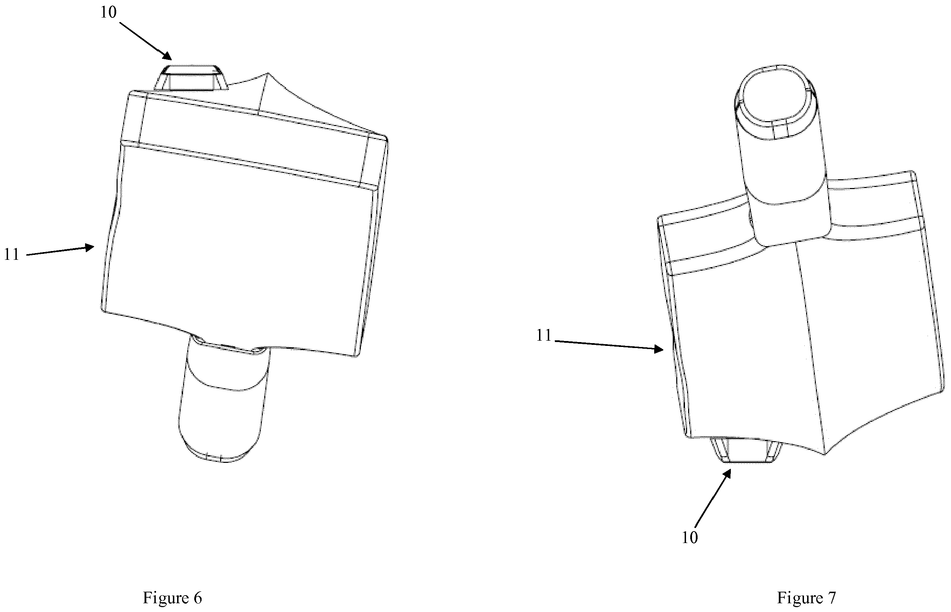

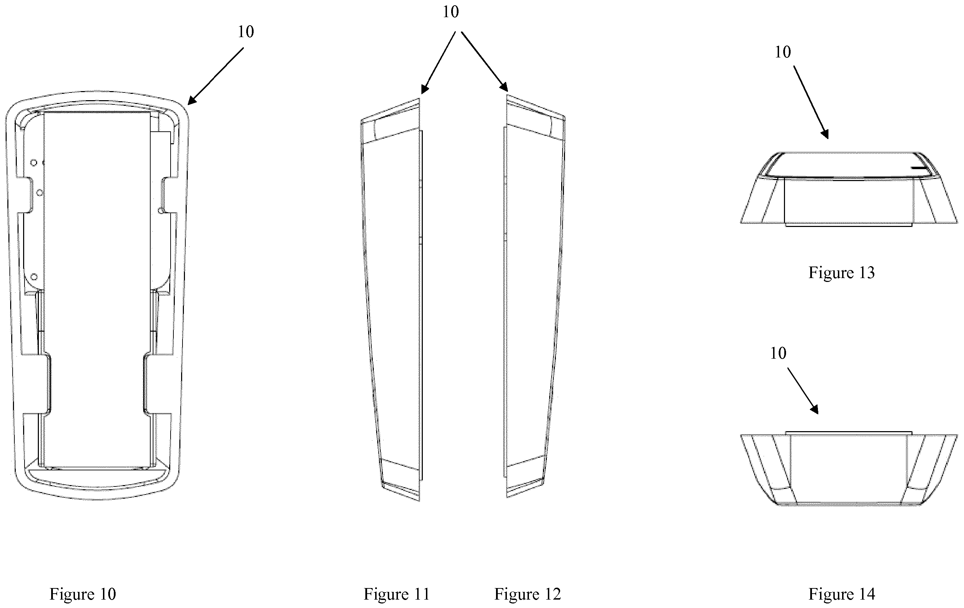

The system of the preferred embodiment includes an inertial measurement unit 10, a preferred embodiment of which and the preferred location relative to a cricket bat as an example, is illustrated in Figures 1 to 14. The illustrated inertial measurement unit 10 includes at least one accelerometer to collect accelerometer raw data, at least one gyroscope to collect gyroscope raw data, at least one transmitter to transmit the accelerometer raw data and the gyroscope raw data, and at least one power source to power the at least one transmitted.

As illustrated, the inertial measurement unit 10 is mounted relative to a cricket bat 11 to collect raw data in relation to the movement of the bat 11 so that the system can return a bat speed, both in general, if the bat 11 is swung and particularly at the instant at which a ball impacts the bat 11 as this information may be useful for training for example to provide to viewers of a match.

The inertial measurement unit 10 of the illustrated embodiment is mounted relative to the bat 11 and the bat 11 is then moved as it is used.

In a particularly preferred embodiment, the inertial measurement unit 10 is mounted relative to the bat close to, if not in all at an impact zone or position. Location in this representative position is particularly important for the accuracy of the calculation of the velocity as it will normally be the velocity of the bat 11 in the representative position which is important. This will typically create issues with the robustness of the inertial measurement unit 10 in order to withstand greater acceleration and/or impacts which may be occasioned in the representative position.

The inertial measurement unit 10 is mounted relative to the bat 11 on the opposite side of the bat to the impact position or hit point as shown in Figures 1 to 7 in particular.

The inertial measurement unit 10 transmits collected raw data over a wireless communication link and the inertial measurement unit is preferably responsible for packetizing the collected raw data prior to transmission.

The collected raw data will undergo some preliminary manipulation prior to transmission in the system of the preferred embodiment. For example, the collected raw data is typically manipulated to compensate for any drift which may occur in any one or more of the data collection components of the inertial measurement unit 10, prior to transmission.

As will be explained further below, it is preferred that the raw data is collected on multiple axes (X, Y, Z).

The raw data (which includes the data which has been manipulated for compensation of drift) is typically sent or transmitted from the inertial measurement unit 10 on a radio frequency but any frequency (including a non-radio frequency) could be used. A particularly preferred embodiment of the present invention transmits on approximately 900 MHz.

The raw data is typically collected over a number of time steps or frames. Any length of time step or frame can be used. A particularly preferred frequency of collection of raw data is preferably approximately 1000 Hz.

The inertial measurement unit 10 includes at least one accelerometer but a single multiaxis accelerometer is preferred.

The inertial measurement unit of the preferred embodiment includes at least one gyroscope but a single multiaxis gyroscope is preferred.

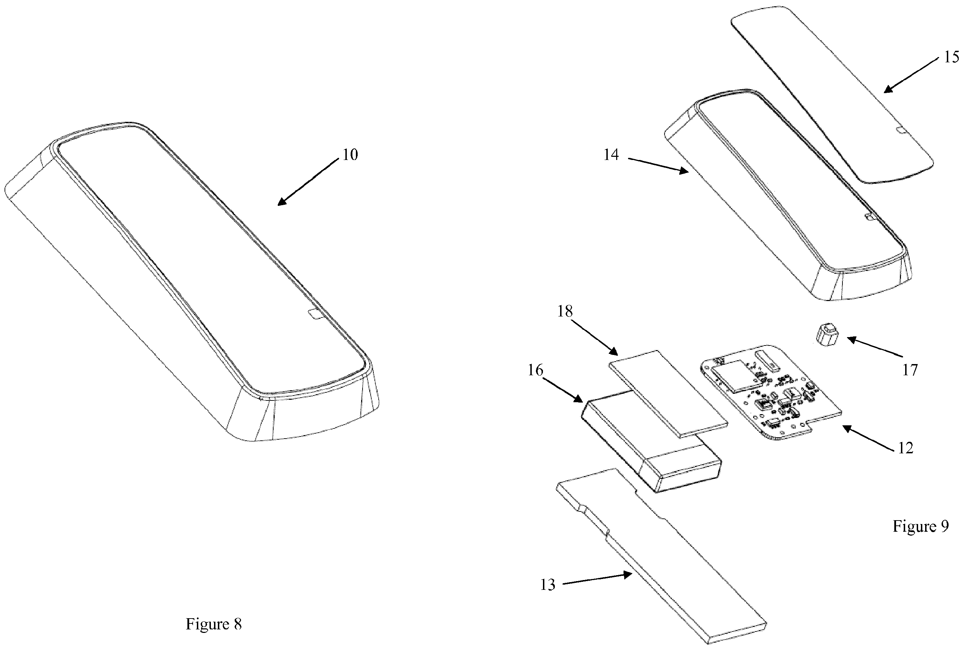

The accelerometer and gyroscope are both provided on or relative to a printed circuit board assembly 12 as shown in Figure 9.

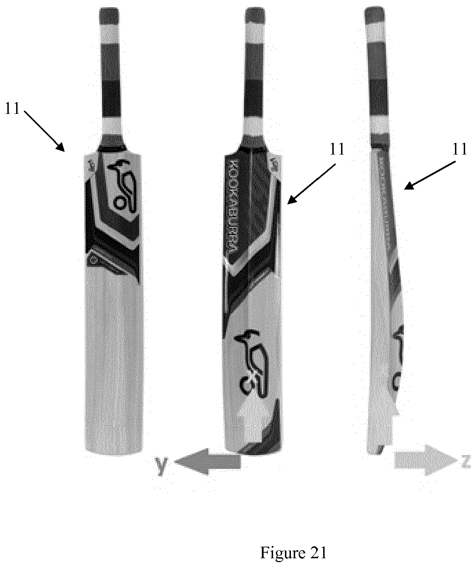

The accelerometer will preferably detect and measure linear acceleration and the gyroscope will preferably detect and measure angular acceleration. Detection and measurement will normally occur relative to at least three axes, wherein a first axis is orthogonal to a second axis which are both orthogonal to a third axis. An example axis system for a cricket bat 11 is shown in Figure 21.

A MEMS (microelectrical-mechanical system) gyroscope is a device that is used for measuring orientation and is a preferred gyroscope according to the present invention.

Single- and multi-axis models of accelerometer are available to detect magnitude and direction of the acceleration, as a vector quantity, and can be used to sense orientation (because direction of weight changes), coordinate acceleration, vibration, shock. Micro-machined microelectrical-mechanical systems (MEMS) accelerometers are increasingly preferred to detect the position of the device, the acceleration of the object or collect data for further processing.

Together the at least one accelerometer and the at least one gyroscope will preferably provide 6 component motion sensing; acceleration for X, Y, and Z movement, both angular acceleration and linear acceleration.

The inertial measurement unit 10 of the preferred embodiment also includes at least one transmitter. As mentioned above, it is preferred that the transmitter is a wireless transmitter and preferably, the transmitter will transmit on a radio frequency. The at least one transmitter is preferably associated with the printed circuit board assembly 12, in order to receive raw data from the accelerometer and gyroscope.

The printed circuit board assembly (PCBA) 12 of the illustrated embodiment also mounts a microcontroller or processor in order to control operation of the components of the inertial measurement unit 10 as well as to apply the compensation for drift prior to transmission of the raw data.

The inertial measurement unit 10 includes an on board battery 16 separated in the inertial measurement unit 10 from the PCBA 12 by a separator 18. The battery 16 is relatively small in size, typically rechargeable, and preferably rechargeable in situ (although removal may be possible if this is preferred). A particularly preferred type of battery is a lithium polymer battery or more correctly, at least one lithium ion polymer battery. The battery may be charged by any mechanism but a wireless form of charging such as induction charging is preferred as this means that the housing can remain substantially sealed.

The inertial measurement unit 10 includes an indicator LED provided on or relative to the printed circuit board assembly 12 in order to indicate the operational status of the inertial measurement unit 10 and/or any one or more of the components of the inertial measurement unit 10. A light pipe 17 or similar structure is provided relative to the indicator LED and the housing of the inertial measurement unit 10 in order to transmit the light to the surface of the housing in order to be visible.

At least one switch is typically provided in relation to the inertial measurement unit to turn the unit on and off. Any type of switch may be used. Although a physical switch could be used, it is preferred that the switch be located inside the housing and operated using a magnet or similar which will mean that the housing can then be a closed housing.

As illustrated in Figures 8 to 14 in particular, the inertial measurement unit 10 is provided with an external housing to enclose the components of the inertial measurement unit 10. As illustrated, the housing is a two part housing including a base plate 13, relative to which the printed circuit board assembly 12 is provided and a cover 14. The housing is manufactured from a robust material and a plastic material is particularly preferred. The housing will normally be provided with a depression in the cover to allow branding 15 to be applied to the exterior of the housing in order to allow the inertial measurement unit 10 to be branded appropriately for the manufacturer or provider of the inertial measurement unit, and/or have team colours or branding applied thereto.

The inertial measurement unit 10 is attached to the bat 11 which may be moved at a relatively high velocity and/or undergo large impacts. For this reason, the attachment mechanism used to attach the inertial measurement unit 10 relative to the bat 11 needs to be extremely robust. Any type of attachment mechanism may be used. In the simplest form, an adhesive strip may be used to attach the inertial measurement unit 10 directly to the bat 11. If an adhesive strip is used, will normally be double sided and preferably thick enough to adjust to any curvature of the object if necessary.

Due to the location of the inertial measurement unit 10 relative to the bat 11 in the illustrated embodiment, the inertial measurement unit 10 will be subjected to large forces, particularly due to impact. Unless precautions are taken, these large forces can result in the detachment of components from the printed circuit board assembly 12 which results in loss of function for the inertial measurement unit 10. Therefore, the inertial measurement unit 10 of the illustrated embodiment includes a foamed material or other resilient padding provided between the housing and the printed circuit board assembly 12 and/or any components mounted to the printed circuit board assembly 12 and particularly between the battery and the enclosure

In a particularly preferred embodiment, the printed circuit board assembly 12 and the components mounted thereto are potted (the process of filling in an assembly with a solid or gelatinous compound for resistance to shock or vibration). Any one or more thermosetting plastics or silicone rubber gels may be used for the potting.

The system of the present invention also includes a raw data processor to receive the accelerometer raw data and the gyroscope raw data from the inertial measurement unit 10, process the accelerometer raw data and the gyroscope raw data to detect at least one movement event of the bat 11 to which the inertial measurement unit 10 is mounted so that when a movement event is detected, a velocity of the moving bat 11 can be returned as a result.

As illustrated in Figures 1 to 7, the inertial measurement unit 10 is mounted to the bat 11 at or immediately adjacent to an impact zone rather than remotely from the impact zone. Locating the inertial measurement unit 10 remotely from the impact zone, such as at the handle for example, requires calculation of the velocity at the impact zone by extrapolating parameters such as the angle, acceleration, and distance to the impact zone based on the raw data which introduces variables or calculation errors due to assumptions. For example, it is very difficult to ascertain the exact impact or hit point of a ball on the bat 11 using an inertial measurements unit mounted to the handle of the bat 11.

In a preferred form, the velocity and collision detection system and method will include the raw data processor undertaking a collision detection step wherein a collision is deemed to have occurred if there is a sudden acceleration change above a predetermined threshold between the raw data at a first time step compared to the raw data at an immediately subsequent time step. If a collision is deemed to have occurred, the system and method of the present invention will preferably return the velocity of the bat 11 calculated at the first time step as a collision velocity.

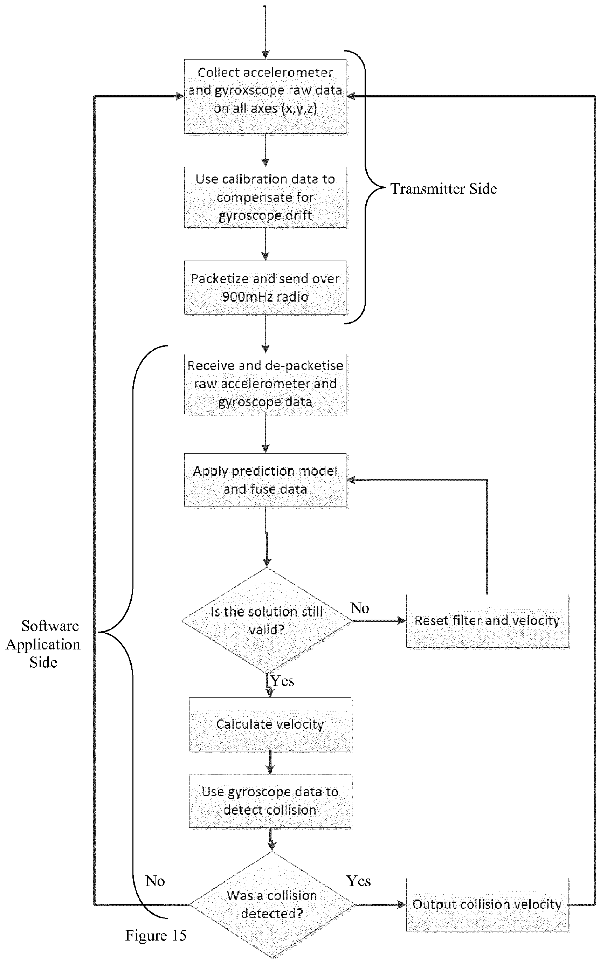

The processor of the preferred embodiment operates according to the flowchart illustrated in Figure 15.

The inertial measurement unit 10 is responsible for collecting the raw data from the components on board the inertial measurement unit (at least one accelerometer, at least on gyroscope and at least one magnetometer), compensate for drift in any one or more of the components, packetize the compensated raw data and transmit the compensated raw data, preferably over a radio frequency such as 900mHz, to a processor to be processed further.

To capture the velocity of the bat at impact time, it is necessary to firstly characterise how a meaningful impact can be detected during a cricket game using the IMU pre-processed inertial data. Pre-processed in this case means the acceleration and gyroscope data have been filtered by the velocity acquisition algorithm. It also means any gaps in the raw data would have been filled based on a prediction model.

In order to detect relevant impacts to a ball for example as it strikes a bat 11, the bat 11 moving direction is assessed first. The algorithm will only detect impacts if the bat 11 is moving towards the ball, which means the acceleration must be negative along the z-axis and positive along the x based on the coordinates system.

Once that condition is met, a collision is detected if a sudden acceleration change reaches a heuristically-defined threshold between two samples. The velocity relative to the first sample is then returned, suggesting that was the speed of the bat immediately before the impact. A hysteresis factor is added to the threshold to avoid multiple consecutive detections.

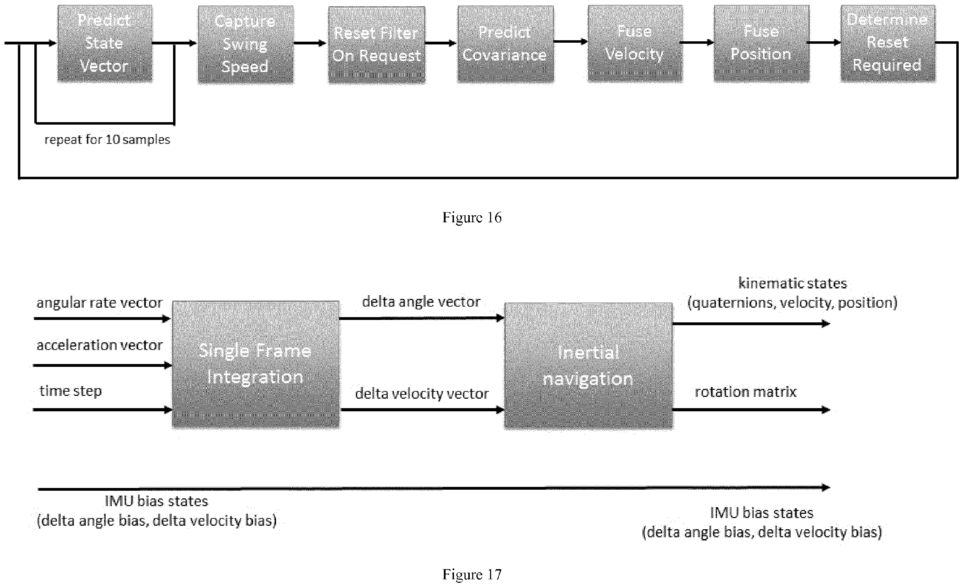

According to the preferred embodiment, the raw data collected by the at least one accelerometer and at least one gyroscope is subjected to a prediction model based on estimation theory. The processes involved in the preferred embodiment are illustrated in Figures 16 to 20.

The raw data collected regarding acceleration will often be associated with one or more random components such as noise for example. A "filter" is used to adjust the raw data to account for the bias of the components in the inertial measurement unit that collect the data for example, the Delta angle bias and the Delta velocity bias.

A preferred filter for use in the preferred embodiment is an Extended Kalman Filter (EKF) which is a non-linear version of a Kalman filter which linear rises about an estimate of the current mean and covariance.

The system of the present invention preferably uses a state vector prediction every time the raw data is received from the inertial measurement unit. The aim with using the state vector prediction model is to predict the state vector containing at least some of:

- the quaternions (4 parameter attitude representation);

- velocity (integrating the acceleration data once will give velocity information);

- position (integrating the acceleration data twice will give position information); and

- inertial measurement unit bias estimates;

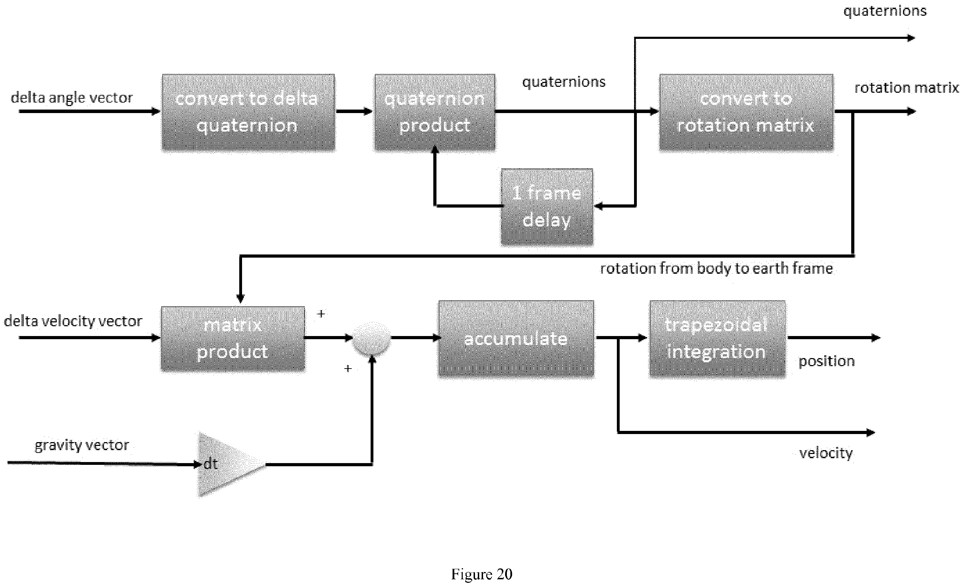

as illustrated in Figure 20.

The system of the present invention uses the state vector prediction model to predict the state vector forward in time using the inertial measurement unit raw data as the input into the model. The implementation of the general model in the preferred embodiment is illustrated in Figure 16. The inertial measurement bias state estimates are preferably not modified in the modelling process but are taken into account.

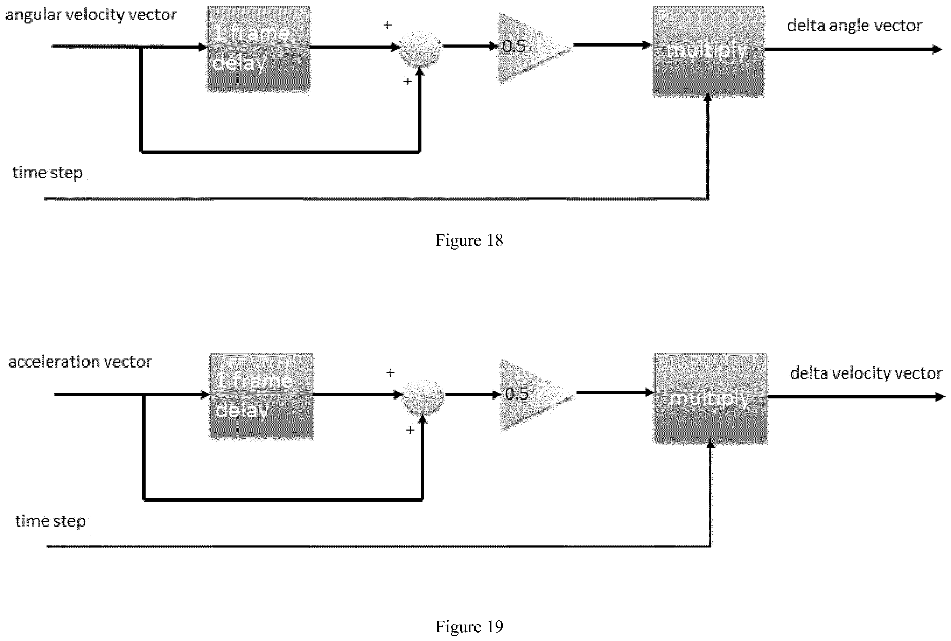

Typically, the process of the preferred embodiment will be used to integrate an angular velocity or rate vector and/or an acceleration vector for each time step or frame in order to calculate a Delta angle vector and the Delta velocity vector which is illustrated in Figure 17 according to the processes illustrated in Figures 18 and 19.

The Delta angle vector can then preferably be converted to a Delta quaternion set to obtain the quaternions.

The quaternions can then preferably be used to convert to a rotation matrix in order to describe the rotation of the object relative to the Earth frame. The rotation matrix can then preferably be used in association with the Delta velocity vector and a gravity vector over time in order to determine velocity information and using trapezoidal integration, to determine position information relating to the object. These processes are illustrated graphically in Figure 20 (which process is the "inertial navigation" box in Figure 17)

The system of the preferred embodiment can then calculate the speed (velocity) of the object as the magnitude of the estimated velocity vector that has been calculated. As shown in Figure 16, this is typically done only one in every ten or so frames (time steps) due to the computational load required to ascertain whether the solution from the prediction model is valid or not. If the solation is not valid the filter and calculated velocity is reset and if the solution is valid, the system then moves to determine whether an impact is detected or not, as shown in Figure 15. If an impact is detected, then the velocity from the time step or frame immediately prior to the impact detection is output as the collision velocity, also as shown in Figure 15.

In further detail, in order to calculate speed as the magnitude of the estimated velocity vector, the system will preferably correct acceleration and angular rate data using learned biases. The system then typically uses the corrected data to calculate the g loading along the object's X and Z axes called 'gx' and 'gz' respectively, and the magnitude of the combined gx and gz magnitude called 'g_level'. The g loading is acceleration divided by gravity. The system then detect object movement events, for example bat swing events using gx and gz to detect the combination of angular and centripetal acceleration associated with that type of motion. The detection of a movement event will normally require g_level to exceed a threshold. A higher g_level threshold can be used if a swing is not declared.

If a swing event is not declared, then a non-swing event is declared. The system then preferably counts the number of swing and non-swing events.

The system will then preferably declare a swing when the required swing event count is exceeded, zero the non-swing event counter and zero the recorded peak speed.

The system will typically cancel the swing declaration when the required non-swing event count is exceeded, zero the swing event counter, set the impact declaration to false and copy the recorded peak speed to the reported peak speed.

If a swing is declared and g_level exceeds a threshold, the system will declare an impact and will output the velocity from the time step or frame immediately prior to the impact detection as the collision velocity.

When a swing is declared and no impact has been declared, the system will normally record the peak speed and may output this peak speed.

As mentioned above, zeroing to correct for drift is desirable if possible. The system of the present invention will preferably reset the filter is the object is still enough to reset the filter. Preferably the system will correct the acceleration and angular rate data using learned biases. The system will then preferably use the magnitude of the angular rate and acceleration vector to perform a motion check and detect if the object is still enough to reset the filter. If the check fails, the system will normally exit the reset and not clear the 'filter reset required' status. If the check passes, the system will normally clear the 'filter reset required' status and continue.

If a bias reset has been requested according to the above check, the system will preferably zero the IMU 10 delta angle and delta velocity bias states. This is normally only requested during the first filter cycle.

The system will then preferably use the measured acceleration estimate the direction of gravity in the object's frame of reference and use that to set the initial angular orientation assuming zero yaw. The system also sets the velocity and position states to zero.

If no bias reset has been requested, according to the above check, the system will preferably record the variances for the IMU delta angle and delta velocity bias states. The system then preferably zeros the covariance matrix.

The system then preferably sets the variances to parameter specified values. If no bias reset has been requested, the system will preferably set the variances for the delta angle and delta velocity states to the value prior to zeroing the covariance matrix.

The estimated growth in state error over time is preferably performed by the 'covariance prediction' operation which uses a non-additive process noise. A number of tuneable parameters are normally provided to control the growth in covariance between measurements. These tuneable parameters include the IMU measurement noise and IMU bias process noise.

When the magnitude of the acceleration vector is below a threshold for a minimum continuous time interval, a zero velocity and zero position assumption is preferably used to prevent the inertial navigation from drifting. Different acceleration and time interval thresholds are normally used for position and velocity. These use a zero velocity or position as an observation vector in the standard EKF state and covariance update equations used in the system calculations. Each axis of the observation vector is typically treated as a separate measurement using a technique called 'sequential fusion' to improve computational efficiency.

A statistical confidence check is applied to each measurement axis and if any axis fails, a test fail is preferably reported and the measurement vector is not used.

If the fuse velocity or fuse position reports a continuous fail for longer than a specified interval, the acceleration magnitude is within specified limits and velocity fusion is active, then a filter reset is typically requested. This enables the filter to recover from the loss of navigation caused by the large acceleration events associated with impact, for example with the ground or a ball.

If the prediction model solution is valid after the statistical confidence check is applied to each measurement axis, then the velocity is calculated.

The system also moves to determine whether an impact has been declared. If an impact has been declared, then the velocity at the immediately preceding frame or time step is output as the collision velocity.

The state vector prediction of the preferred embodiment runs every time IMU 10 data is received (1000 Hz), however the follow-on processes are only performed every tenth frame. This is done because the covariance prediction and measurement fusion require a significant amount of processing.

This function predicts the state vector containing the quaternions (4 parameter attitude representation), velocity, position, and IMU bias estimates forward in time using the IMU input data. IMU bias state estimates are not modified by this process.

Firstly, a single state integration is undertaken using the angular rate vector, acceleration vector and time step from the IMU to calculate a delta angle vector as illustrated in Figure 18 and a delta velocity vector illustrated in Figure 19 which are then used as inputs into the inertial navigation calculation, illustrated in Figure 20 to predict the quaternions (4 parameter attitude representation), velocity, and position.

Further explanation as to the function of each process shown in Figure 16 after the prediction of eth stat vector is as follows:

Capture Swing Speed

1. Calculate speed as the magnitude of the estimated velocity vector
2. Correct acceleration and angular rate data using learned biases
3. Use corrected data to calculate the the g loading along the bats X and Z axes called 'gx' and 'gz' respectively, and the magnitude of the combined gx and gz magnitude called 'g_level'. The g loading is acceleration divided by gravity.
4. Detect bat swing events using gx and gz to detect the combination of angular and centripetal acceleration associated with that type of motion. Require g_level to exceed a threshold. Use a higher g_level threshold if a swing is not declared.
5. If a swing event is not declared, then a non-swing event is declared.
6. Count the number of swing and non-swing events.
7. Declare a swing when the required swing event count is exceeded, zero the non-swing event counter and zero the recorded peak speed.
8. Cancel the swing declaration when the required non-swing event count is exceeded, zero the swing event counter, set the impact declaration to false and copy the recorded peak speed to the reported peak speed.
9. If swinging is declared and g_level exceeds a threshold, declare an impact.
10. When a swing is declared and no impact has been declared, record the peak speed.

Reset Filter on Request

1. Correct acceleration and angular rate data using learned biases
2. Use the magnitude of the angular rate and acceleration vector to perform a motion check and detect if the bat is still enough to reset the filter. If the check fails, exit the reset and do not clear the 'filter reset required' status. If the check passes, clear the 'filter reset required' status and continue.
3. If a bias reset has been requested, zero the IMU delta angle and delta velocity bias states. This is only requested during the first filter cycle.
4. Use the measured acceleration estimate the direction of gravity in the bats frame of reference and use that to set the initial angular orientation assuming zero yaw.
5. Set the velocity and position states to zero.
6. If no bias reset has been requested, record the variances for the IMU delta angle and delta velocity bias states.
7. Zero the covariance matrix.
8. Set the variances to parameter specified values. If no bias reset has been requested, set variances for the delta angle and delta velocity states to the value prior zeroing the covariance matrix.

Predict Covariance

The estimated growth in state error over time is performed by the 'covariance prediction' operation which uses a non-additive process noise. A number of tuneable parameters are provided to control the growth in covariance between measurements.

$$P_k = F_{k-1} P_{k-1} F_{k-1}^T + G_{k-1} Q_{k-1} G_{k-1}^T + Q_s$$

where

$P_k$ is the state covariance matrix;
$Q_{k-1}$ is the IMU measurement noise covariance matrix; and
$Q_s$ is the IMU bias process noise covariance matrix.

The state and control partial derivative matrices used in the above equation are:

$$F_k = \left(\frac{\partial f}{\partial x}\right)_k$$

$$G_k = \left(\frac{\partial f}{\partial u}\right)_k$$

The state covariance matrix has symmetry forced and variances (diagonals) are constrained to be non-negative to prevent filter failure due to cumulative numerical errors.

Fuse Velocity and Fuse Position

1. When the magnitude of the acceleration vector is below a threshold for a minimum continuous time interval, a zero velocity and zero position assumption is used to prevent the inertial navigation from drifting.
2. Different acceleration and time interval thresholds used for position and velocity.
3. These use a zero velocity or position as an observation vector in the standard EKF state and covariance update equations (see next slide)
4. Each axis of the observation vector is treated as a separate measurement using a technique called 'sequential fusion' to improve computational efficiency.
5. A statistical confidence check is applied to each measurement axis and if any axis fails, a test fail is reported and the measurement vector is not used.

EKF Measurement Fusion

This updates the state estimates and covariance matrix using measurements. The covariance values will always decrease after measurements are fused because new information is gained.

Observation Jacobian function: $H_k = \left(\dfrac{\partial z_p}{\partial x}\right)_k$

Innovation Variance: $S_k = H_k P_k^- H_k^T + R_k$

Where $P_k^-$ is the state covariance matrix; and
Rk is the observation covariance.

Kalman Gain: $K = P_k^- H_k S_k^{-1}$

Innovation:

$v = z - z_p$

Where *z* is an actual measurement generally zero in the present application; and
$z_p$ is the measurement predicted using the state vector

Covariance Update: $P_k^+ = \begin{bmatrix} I - KH_k \end{bmatrix} P_k^-$

State Update: $x_k^+ = x_k^- + K\nu$

Determine Reset Required

1. If the fuse velocity or fuse position report a continuous fail for longer than a specified interval, the acceleration magnitude is within specified limits and velocity fusion is active, then a filter reset is requested.

2. This enables the filter to recover from the loss of navigation caused by the large acceleration events associated with ground or ball impact

The states at the current time step are expressed as a function of the states at the previous time step and known system inputs (IMU data). The equations that do this are referred to as the 'state equations':

$$x_k = f\left(x_{k-1}, u_k\right)$$

$x_k$: predicted states at current time-step
$x_{k-1}$: states from previous time-step
$u_k$: known system inputs at current time step

Pose information is captured in the first 10 states which use a dynamic process model that defines the movement of the bat frame (XYZ RH axis system) in a

$$\begin{bmatrix} q_0 \\ q_1 \\ q_2 \\ q_3 \\ V_X \\ V_Y \\ V_D \\ P_X \\ P_Y \\ P_D \end{bmatrix}$$

Quaternion rotation from nav to bat frame

X, Y, Down velocity

X, Y, Down position

navigation inertial reference frame (X, Y, Down RH axis system)

The first four states are the quaternions that define the angular position of the XYZ bat frame relative to XYD navigation frame.

$$\begin{bmatrix} q_0 \\ q_1 \\ q_2 \\ q_3 \end{bmatrix}$$

The rotation matrix from bat to navigation frame is given by:

$$\left. \begin{array}{c} \\ \\ \\ \end{array} \right\} \quad \text{X, Y, Down position}$$

$$[T]_B^N = \begin{bmatrix} q_0^2 + q_1^2 - q_2^2 - q_3^2 & 2\left(q_1 \cdot q_2 - q_0 \cdot q_3\right) & 2\left(q_1 \cdot q_3 + q_0 \cdot q_2\right) \\ 2\left(q_1 \cdot q_2 + q_0 \cdot q_3\right) & q_0^2 - q_1^2 + q_2^2 - q_3^2 & 2\left(q_2 \cdot q_3 - q_0 \cdot q_1\right) \\ 2\left(q_1 \cdot q_3 - q_0 \cdot q_2\right) & 2\left(q_2 \cdot q_3 + q_0 \cdot q_1\right) & q_0^2 - q_1^2 - q_2^2 + q_3^2 \end{bmatrix}$$

Where the rotation from navigation to bat frame is required, the transpose will be used and is denoted by $[T]_N^B$

The truth delta angles are calculated from the IMU measurements and delta angle bias states $\Delta_{ang\_bias}$.

$$\Delta_{ang\_meas} = \begin{bmatrix} \Delta_{ang\_x} \\ \Delta_{ang\_y} \\ \Delta_{ang\_z} \end{bmatrix} = \int_{t_k}^{t_{k+1}} \omega \cdot dt$$

$$\Delta_{ang\_bias} = \begin{bmatrix} \Delta_{ang\_bias\_x} \\ \Delta_{ang\_bias\_y} \\ \Delta_{ang\_bias\_z} \end{bmatrix} \quad \text{Delta angle bias states}$$

$$\Delta_{ang\_truth} = \Delta_{ang\_meas} - \Delta_{ang\_bias}$$

The truth delta velocities are calculated from the IMU measurements and delta velocity states $\Delta_{vel\_bias}$.

$$\Delta_{vel\_meas} = \begin{bmatrix} \Delta_{vel\_x} \\ \Delta_{vel\_y} \\ \Delta_{vel\_z} \end{bmatrix} \quad \text{Delta angle IMU measurements}$$

$$\Delta_{vel\_bias} = \begin{bmatrix} \Delta_{vel\_bias\_x} \\ \Delta_{vel\_bias\_y} \\ \Delta_{vel\_bias\_z} \end{bmatrix} \quad \text{Delta angle bias states}$$

$$\Delta_{vel\_truth} = \Delta_{vel\_meas} - \Delta_{vel\_bias}$$

The quaternion $\Delta_{quat}$ that defines the rotation from the quaternion at frame k to k+1 is calculated from the truth delta angle $\Delta_{ang\_truth}$ using a small angle approximation. The inertial navigation prediction in the filter uses an exact method.

$$\Delta_{quat} = \begin{bmatrix} \Delta q_0 \\ \Delta q_1 \\ \Delta q_2 \\ \Delta q_3 \end{bmatrix} = \begin{bmatrix} 1 \\ \dfrac{\Delta_{ang\_truth\_x}}{2} \\ \dfrac{\Delta_{ang\_truth\_y}}{2} \\ \dfrac{\Delta_{ang\_truth\_z}}{2} \end{bmatrix}$$

The quaternion product rule is used to rotate the quaternion state forward by the delta quaternion $\Delta_{quat}$ from frame k to k+1.

$$\begin{bmatrix} q_0 \\ q_1 \\ q_2 \\ q_3 \end{bmatrix}_{k+1} = \begin{bmatrix} q_0 \Delta q_0 - q_1 \Delta q_1 - q_2 \Delta q_2 - q_3 \Delta q_3 \\ q_0 \Delta q_1 + \Delta q_0 q_1 + q_2 \Delta q_3 - \Delta q_2 q_3 \\ q_0 \Delta q_2 + \Delta q_0 q_2 - q_1 \Delta q_3 + \Delta q_1 q_3 \\ q_0 \Delta q_3 + \Delta q_0 q_3 + q_1 \Delta q_2 - \Delta q_1 q_2 \end{bmatrix}$$

The truth delta velocity vector is rotated from bat frame to navigation frame and gravity is subtracted to calculate the change in velocity states from frame k to k+1.

$$
\begin{bmatrix} V_X \\ V_Y \\ V_D \end{bmatrix}_{k+1} =
$$

$$
\begin{bmatrix} V_X \\ V_Y \\ V_D \end{bmatrix}_k + [T]_B^N \cdot \Delta_{vel\_truth} + \begin{bmatrix} 0 \\ 0 \\ g \end{bmatrix} \cdot \Delta t
$$

The position states are updated using Euler integration (the inertial navigation uses a more accurate trapezoidal integration method).

$$
\begin{bmatrix} P_X \\ P_Y \\ P_D \end{bmatrix}_{k+1} = \begin{bmatrix} P_X \\ P_Y \\ P_D \end{bmatrix}_k + \begin{bmatrix} V_X \\ V_Y \\ V_D \end{bmatrix}_k \cdot \Delta
$$

IMU sensor bias uses a static process model where we assume that they are nominally constant with state prediction noise allowing them to change slowly. These are represented by states 11 to 16, eg

$$
\begin{bmatrix} \Delta_{ang\_bias\_x} \\ \Delta_{ang\_bias\_y} \\ \Delta_{ang\_bias\_z} \\ \Delta_{vel\_bias\_x} \\ \Delta_{vel\_bias\_y} \\ \Delta_{vel\_bias\_z} \end{bmatrix}_{k+1} = \begin{bmatrix} \Delta_{ang\_bias\_x} \\ \Delta_{ang\_bias\_y} \\ \Delta_{ang\_bias\_z} \\ \Delta_{vel\_bias\_x} \\ \Delta_{vel\_bias\_y} \\ \Delta_{vel\_bias\_z} \end{bmatrix}_k
$$

$\left.\rule{0pt}{2.5em}\right\}$ IMU delta angle bias

$\left.\rule{0pt}{2.5em}\right\}$ IMU delta velocity bias

In the present specification and claims (if any), the word 'comprising' and its derivatives including 'comprises' and 'comprise' include each of the stated integers but does not exclude the inclusion of one or more further integers.

Reference throughout this specification to 'one embodiment' or 'an embodiment' means that a particular feature, structure, or characteristic described in connection with the embodiment is included in at least one embodiment of the present invention. Thus, the appearance of the phrases 'in one embodiment' or 'in an embodiment' in various places throughout this specification are not necessarily all referring to the same embodiment. Furthermore, the particular features, structures, or characteristics may be combined in any suitable manner in one or more combinations.

In compliance with the statute, the invention has been described in language more or less specific to structural or methodical features. It is to be understood that the invention is not limited to specific features shown or described since the means herein described comprises preferred forms of putting the invention into effect. The invention is, therefore, claimed in any of its forms or modifications within the proper scope of the appended claims (if any) appropriately interpreted by those skilled in the art.

## Claims

1. A velocity and collision detection system including

   a) an inertial measurement unit having at least one accelerometer to collect accelerometer raw data, at least one gyroscope to collect gyroscope raw data, at least one transmitter to transmit the accelerometer raw data and the gyroscope raw data, and at least one power source to power the at least one transmitter,

   the inertial measurement unit configured to be mounted relative to a moving object to collect raw data in relation to the moving object, and

   b) a raw data processor to receive the accelerometer raw data and the gyroscope raw data from the inertial measurement unit, process the accelerometer raw data and the gyroscope raw data to detect at least one movement event of the object to which the inertial measurement unit is mounted so that when a movement event is detected, at least one movement parameter of the moving object is calculated.

2. The velocity and collision detection system as claimed in claim 1 further including the raw data processor undertaking a collision detection step wherein a collision is deemed to have occurred if there is a sudden acceleration change above a predetermined threshold between the raw data at a first time step compared to the raw data at an immediately subsequent time step.

3. The velocity and collision detection system as claimed in claim 2 wherein if a collision is deemed to have occurred, the system returns the velocity of the object calculated at the first time step as a collision velocity.

4. The system as claimed in any one of the preceding claims wherein the at least one movement parameter is or includes a parameter relating to the movement of the object, including real time acceleration, real time angular acceleration, real time velocity and/or real time angular velocity or components of any one of the aforementioned in any one or more axes.

5. The system as claimed in any one of the preceding claims wherein the at least one movement parameter includes an angular measurement or deviation.

6. The system as claimed in any one of the preceding claims wherein the collected raw data is adjusted prior to transmission to compensate for any drift which may occur in the inertial measurement unit prior to transmission.

7. The system as claimed in any one of the preceding claims wherein the raw data is collected on multiple axes (X, Y, Z).

8. The system as claimed in any one of the preceding claims wherein the raw data is collected over a number of time steps or frames.

9. An inertial measurement unit for use in a velocity and collision detection system, the inertial measurement unit having at least one accelerometer to collect accelerometer raw data, at least one gyroscope to collect gyroscope raw data, at least one transmitter to transmit the accelerometer raw data and the gyroscope raw data over a wireless communication pathway, and at least one power source to power the at least one transmitter, the inertial measurement unit configured to be mounted relative to a moving object to collect raw data in relation to the moving object.

**10.** An inertial measurement unit as claimed in claim 9 mounted to the moving object at or immediately adjacent to point of interest such as an impact zone or point of movement.

**11.** The inertial measurement unit as claimed in claim 9 wherein together the at least one accelerometer and the at least one gyroscope provide six component motion sensing namely angular acceleration and linear acceleration for each of X, Y, and Z axes movement.

**12.** The inertial measurement unit as claimed in claim 9 further including at least one magnetometer to assist with ascertaining the direction or heading of the object and/or its movement.

**13.** The inertial measurement unit as claimed in claim 9 wherein the at least one gyroscope collects data in relation to angular acceleration and the at least one accelerometer collects data in relation to linear acceleration.

**14.** A velocity and collision detection method including

a) Providing an inertial measurement unit having at least one accelerometer to collect accelerometer raw data, at least one gyroscope to collect gyroscope raw data, at least one transmitter to transmit the accelerometer raw data and the gyroscope raw data, and at least one power source to power the at least one transmitter,
b) Mounting the inertial measurement unit relative to a moving object to collect raw data in relation to the moving object,
c) receiving the accelerometer raw data and the gyroscope raw data from the inertial measurement unit at a raw data processor,
d) processing the accelerometer raw data and the gyroscope raw data to detect at least one movement event of the object to which the inertial measurement unit is mounted, and
e) calculating at least one movement parameter of the moving object when a movement event is detected.

**15.** The velocity and collision detection method as claimed in claim 19 further including the raw data processor undertaking a collision detection step wherein a collision is deemed to have occurred if there is a sudden acceleration change above a predetermined threshold between the raw data at a first time step compared to the raw data at an immediately subsequent time step.

**16.** The velocity and collision detection method as claimed in claim 20 wherein if a collision is deemed to have occurred, the system returns the velocity of the object calculated at the first time step as a collision velocity.

Figure 2

Figure 1

Figure 5

Figure 4

Figure 3

Figure 7

Figure 6

EP 3 511 722 A1

Figure 9

Figure 8

13

12

17

16

18

14

15

10

Figure 13

Figure 14

Figure 12

Figure 11

Figure 10

Figure 15

Figure 16

Figure 17

angular velocity vector

1 frame delay

+
+

0.5

multiply

delta angle vector

time step

Figure 18

EP 3 511 722 A1

acceleration vector

1 frame delay

+
+

0.5

multiply

delta velocity vector

time step

Figure 19

Figure 20

EP 3 511 722 A1

Figure 21

**Europäisches Patentamt**
**European Patent Office**
**Office européen des brevets**

# EUROPEAN SEARCH REPORT

Application Number

EP 18 21 0193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/057112 A1 (SAK LAVIE [US] ET AL) 26 February 2015 (2015-02-26) * page 1, paragraph 25 - page 5, paragraph 70; figures 1-3 * | 1-16 | INV. G01P15/08 G01P15/04 A63B60/46 |
| X | WO 02/35184 A2 (FIBERSENSE TECHNOLOGY CORP [US]; PERLMUTTER MICHAEL S [US]) 2 May 2002 (2002-05-02) * page 8, line 18 - page 15, line 2; figures 1-3 * | 1-11, 13-16 | |
| X | US 2014/288874 A1 (MATSUNAGA HIDEYUKI [JP] ET AL) 25 September 2014 (2014-09-25) * page 2, paragraph 53 - page 7, paragraph 102; figures 1-9 * | 1-16 | |
| X | US 2017/095691 A1 (ONUKI MASAHIDE [JP]) 6 April 2017 (2017-04-06) * page 2, paragraph 4 - page 4, paragraph 60 * * page 7, paragraph 73 - paragraph 79; figures 1-5 * | 1-16 | |
| X A | US 2015/120021 A1 (KERHUEL LUBIN [FR] ET AL) 30 April 2015 (2015-04-30) * page 4, paragraph 96 - page 6, paragraph 123; figures 1-4 * | 1-11, 14-16 12,13 | TECHNICAL FIELDS SEARCHED (IPC) G01P A63B |
| X A | US 2015/224380 A1 (IWATA MOTOTAKA [JP] ET AL) 13 August 2015 (2015-08-13) * page 2, paragraph 48 - page 3, paragraph 55; figures 1-4 * | 1-11, 14-16 12,13 | |

The present search report has been drawn up for all claims

| Place of search | Date of completion of the search | Examiner |
|---|---|---|
| Munich | 8 May 2019 | Springer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 21 0193

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2019

| Patent document cited in search report | | Publication date | Patent family member(s) | | Publication date |
|---|---|---|---|---|---|
| US 2015057112 | A1 | 26-02-2015 | NONE | | |
| WO 0235184 | A2 | 02-05-2002 | AU | 1459702 A | 06-05-2002 |
| | | | US | 2002123386 A1 | 05-09-2002 |
| | | | US | 2003207718 A1 | 06-11-2003 |
| | | | WO | 0235184 A2 | 02-05-2002 |
| US 2014288874 | A1 | 25-09-2014 | CN | 104056441 A | 24-09-2014 |
| | | | CN | 204121706 U | 28-01-2015 |
| | | | EP | 2782046 A2 | 24-09-2014 |
| | | | JP | 5803962 B2 | 04-11-2015 |
| | | | JP | 2014183931 A | 02-10-2014 |
| | | | US | 2014288874 A1 | 25-09-2014 |
| US 2017095691 | A1 | 06-04-2017 | JP | 2017070366 A | 13-04-2017 |
| | | | US | 2017095691 A1 | 06-04-2017 |
| US 2015120021 | A1 | 30-04-2015 | EP | 2846883 A1 | 18-03-2015 |
| | | | FR | 2990356 A1 | 15-11-2013 |
| | | | FR | 2990357 A1 | 15-11-2013 |
| | | | US | 2015120021 A1 | 30-04-2015 |
| | | | US | 2018339208 A1 | 29-11-2018 |
| | | | WO | 2013167395 A1 | 14-11-2013 |
| US 2015224380 | A1 | 13-08-2015 | JP | 6027038 B2 | 16-11-2016 |
| | | | JP | 2015150130 A | 24-08-2015 |
| | | | US | 2015224380 A1 | 13-08-2015 |

EPO FORM P0459

For more details about this annex : see Official Journal of the European Patent Office, No. 12/82